(19)

Europäisches
Patentamt
European
Patent Office
Office européen
des brevets

(11) **EP 2 671 575 A1**

(12) **EUROPEAN PATENT APPLICATION**

(43) Date of publication:
**11.12.2013 Bulletin 2013/50**

(51) Int Cl.:
*A61K 31/132* (2006.01)　　　*A61K 35/02* (2006.01)

(21) Application number: **12170715.2**

(22) Date of filing: **04.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Universität Regensburg**
**93053 Regensburg (DE)**

(72) Inventors:
 • **Kalbitzer, Hans Robert**
 **93055 Regensburg (DE)**

 • **Kreitner, Sandra**
 **83629 Weyarn (DE)**
 • **Spörner, Michael**
 **93051 Regensburg (DE)**

(74) Representative: **Engelhard, Markus**
 **Boehmert & Boehmert**
 **Pettenkoferstrasse 20-22**
 **80336 München (DE)**

(54) **Ras inhibitors**

(57)　The present invention relates to compounds which inhibit the protein product Ras of the proto-oncogene ras, more particularly Ras-effector interaction, by stabilizing conformational state 1(T) of Ras GTP having a decreased effector affinity. It further relates to the use of these compounds as medicaments and anti-tumor agents and to pharmaceutical compositions comprising such compounds.

EP 2 671 575 A1

**Description**

The present invention relates to compounds which inhibit the protein product Ras of the proto-oncogene ras, more particularly Ras-effector interaction, by stabilizing conformational state 1(T) of Ras GTP having a decreased effector affinity. It further relates to the use of these compounds as medicaments and anti-tumor agents and to pharmaceutical compositions comprising such compounds.

The guanine nucleotide binding (GNB) protein Ras (<u>Ra</u>t <u>S</u>arcoma; also referred to as p21$^{ras}$) is involved in cellular signal transduction pathways that induce proliferation, differentiation and apoptosis of cells. There are three functional ras genes in mammals/humans, H-ras, K-ras, N-ras, which encode highly similar proteins. Ras functions as a molecular switch, cycling between an inactive GDP-bound state and an active GTP-bound state. Only Ras complexed with GTP (Ras GTP) is able to bind different effectors, such as Raf kinase, RalGDS, Norel, and P13K with high affinity (Wittinghofer and Waldmann, 2000). $^{31}$P NMR spectroscopy has revealed the existence of two distinct conformational states, which are in dynamic equilibrium, when wild-type Ras is bound to GTP or the slowly hydrolysable GTP analogues GppNHp, GppCH$_2$p or GTPγS. Whereas conformational state 2(T) is recognized by effector proteins, conformational state 1(T) shows a more than 20-fold decreased affinity to effector proteins (Geyer et al., 1996; Kalbitzer et al., 2009; Spoemer et al., 2001, 2005a, 2007, 2010). Thus, conformational state 1(T) represents a weak binding state for effectors.

In more than 30% of all human cancers Ras is found to be mutated at amino acid position 12, 13 or 61 (Bos, 1989). In the cell, these mutants are locked in the active GTP-bound state owing to their loss of intrinsic as well as GAP (GTPase activating protein) accelerated GTP hydrolysis, which permanent activity leads to increased cell growth and cell proliferation. It should be possible to interrupt the effector interaction of activated, GTP-bound oncogenic Ras by suitable small compounds that selectively stabilize conformational state 1 (T).

As shown by $^{31}$P NMR spectroscopy and structural analysis, the zinc(II) complex of 1,4,7,10-tetraazacyclodo-decane complex (Zn$^{2+}$-cyclen) selectively binds to conformational state 1(T) of activated Ras (Ras GTP), shifts the dynamic equilibrium of activated Ras towards the weak binding state, and thus inhibits effector binding to Ras GTP (Spoemer et al., 2005b; Rosnizeck et al., 2010; WO 2004/006934). The binding site of this compound responsible for selective stabilization of conformational state 1 (T) with decreased effector affinity is close to the γ-phosphate of the nucleotide in the active center of Ras GTP (Rosnizeck et al., 2010).

The results obtained with Zn$^{2+}$-cyclen demonstrate the general applicability of the underlying inhibition mechanism. However, the use of Zn$^{2+}$-cyclen as an inhibitor has at least two drawbacks: metal compounds, such as Zn$^{2+}$-cyclen, are traditionally not accepted as pharmaceuticals, and the affinity of Zn$^{2+}$-cyclen was too low for pharmaceutical applications.

Accordingly, it was an object of the present invention to provide non-metal-containing compounds that inhibit Ras-effector interaction by selectively stabilizing conformational state 1(T) of Ras GTP. It was another object of the present invention to provide compounds that inhibit Ras-effector interaction with high affinity.

The objects of the present invention are solved by a compound selected from the group consisting of (1S, 2S)-cyclohexane-1,2-diamine, 3-phenyl-1H-pyrazole, (4-(1H-imidazol-1-yl)phenyl)methanol, N-methyl-1-(4-methyl-2-phenyl-1,3-thiazol-5-yl)methanamine, (4-(pyridin-4-yl)phenyl)methanol, (3-(pyridin-4-yl)phenyl)methanol, (4H-thieno [3,2-b]pyrrol-5-yl)-methanol, (2-phenoxyphenyl)methanol, benzene-1,2-diamine, 4-phenylpiperazine-1-carboximidamide, (E)-3-(2-(3,4-dihydronaphthalen-1(2H)-ylidene)hydrazinyl)benzoic acid, N-carbamimidoyl-3,4-dihydroquinoline-1 (2H)-carboximidamide, (3-(1H-pyrrol-1-yl)phenyl)methanamine; 1-(imidazo[1,2-a]pyridin-6-yl)-N-methylmethanamine; N-methyl-1-(1-methyl-3-phenyl-1H-pyrazol-5-yl)methanamine; 5-chloro-2-(methylthio)aniline; 1,3-diphenylthiourea; N-(2-(phenylamino)phenyl)acetamide; N2-methyl-N2-phenylpyridine-2,5-diamine; 5-(2-cyclopentylidenehydrazinyl)-4H-1,2,4-triazole-3-thiol, 2,3-diamino-1-benzyl-1H-benzo[d]imidazol-3-ium; (E)-4-(benzylideneamino)phenol; 3-chloro-N-phenylaniline; 1-(2-chlorophenyl)-3-phenylthiourea; 2-(furan-2-ylmethyl)cyclopentanamine and derivatives, enantiomers, diastereomers and pharmaceutically acceptable salts of any of the foregoing for use as a medicament.

The objects of the present invention are also solved by a compound selected from the group consisting of (1S, 2S)-cyclohexane-1,2-diamine, 3-phenyl-1H-pyrazole, (4-(1H-imidazol-1-yl)phenyl)methanol, N-methyl-1-(4-methyl-2-phenyl-1,3-thiazol-5-yl)methanamine, (4-(pyridin-4-yl)phenyl)methanol, (3-(pyridin-4-yl)phenyl)methanol, (4H-thieno [3,2-b]pyrrol-5-yl)-methanol, (2-phenoxyphenyl)methanol, benzene-1,2-diamine, 4-phenylpiperazine-1-carboximidamide, (E)-3-(2-(3,4-dihydronaphthalen-1(2H)-ylidene)hydrazinyl)benzoic acid, N-carbamimidoyl-3,4-dihydroquinoline-1 (2H)-carboximidamide, (3-(1H-pyrrol-1-yl)phenyl)methanamine; 1-(imidazo[1,2-a]pyridin-6-yl)-N-methylmethanamine; N-methyl-1-(1-methyl-3-phenyl-1H-pyrazol-5-yl)methanamine; 5-chloro-2-(methylthio)aniline; 1,3-diphenylthiourea; N-(2-(phenylamino)phenyl)acetamide; N2-methyl-N2-phenylpyridine-2,5-diamine; 5-(2-cyclopentylidenehydrazinyl)-4H-1,2,4-triazole-3-thiol, 2,3-diamino-1-benzyl-1H-benzo[d]imidazol-3-ium; (E)-4-(benzylideneamino)phenol; 3-chloro-N-phenylaniline; 1-(2-chlorophenyl)-3-phenylthiourea; 2-(furan-2-ylmethyl)cyclopentanamine and derivatives, enantiomers, diastereomers and pharmaceutically acceptable salts of any of the foregoing for use as an anti-tumor agent.

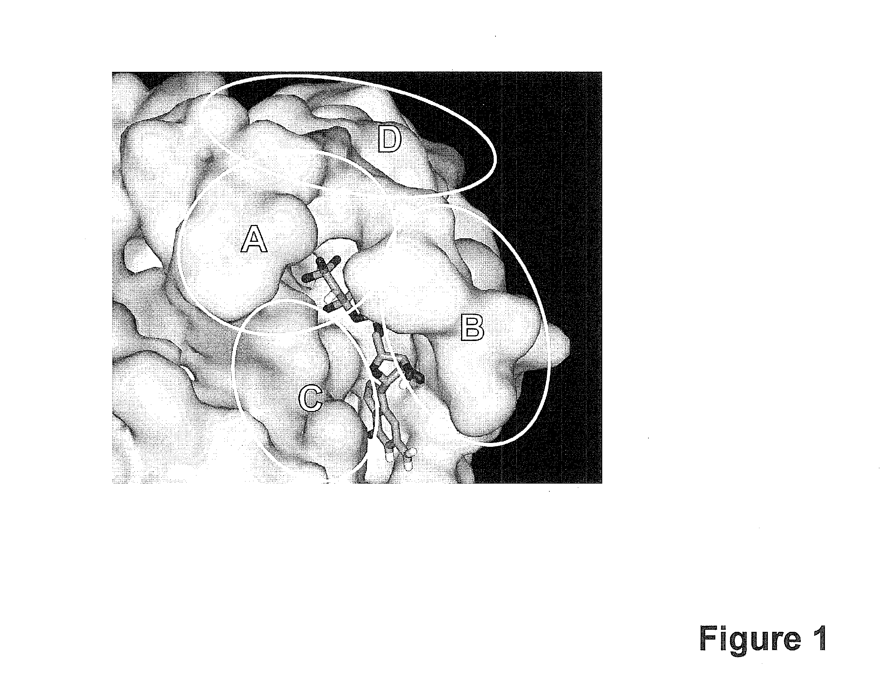

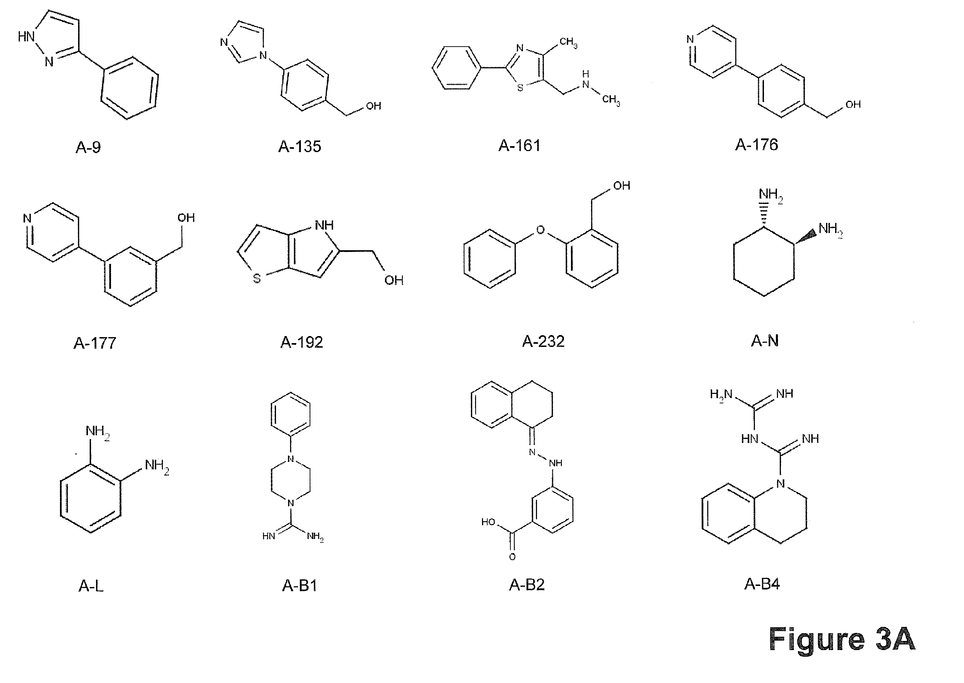

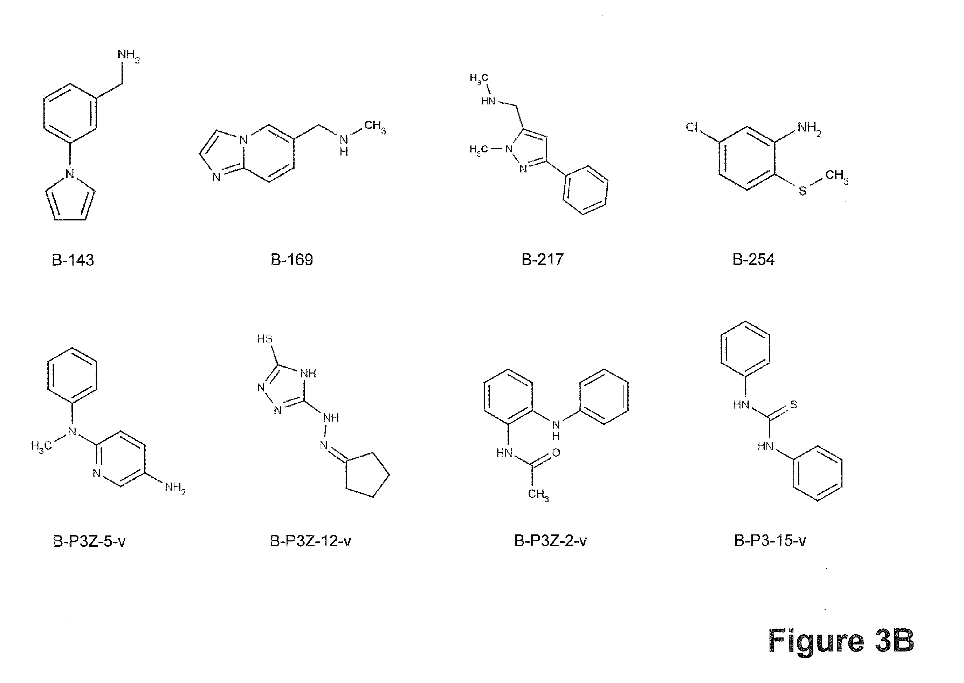

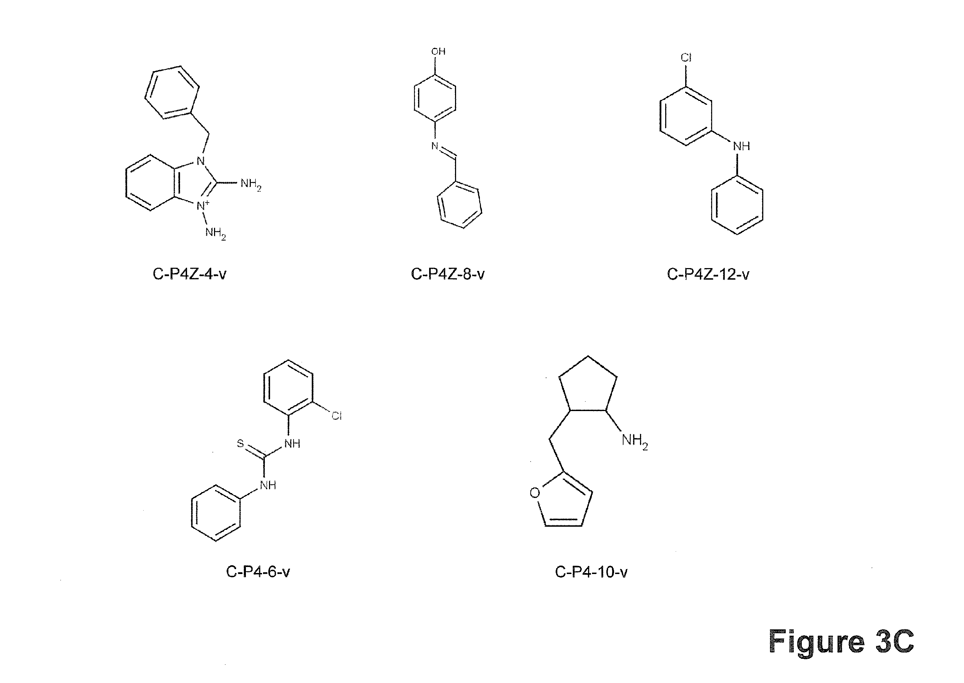

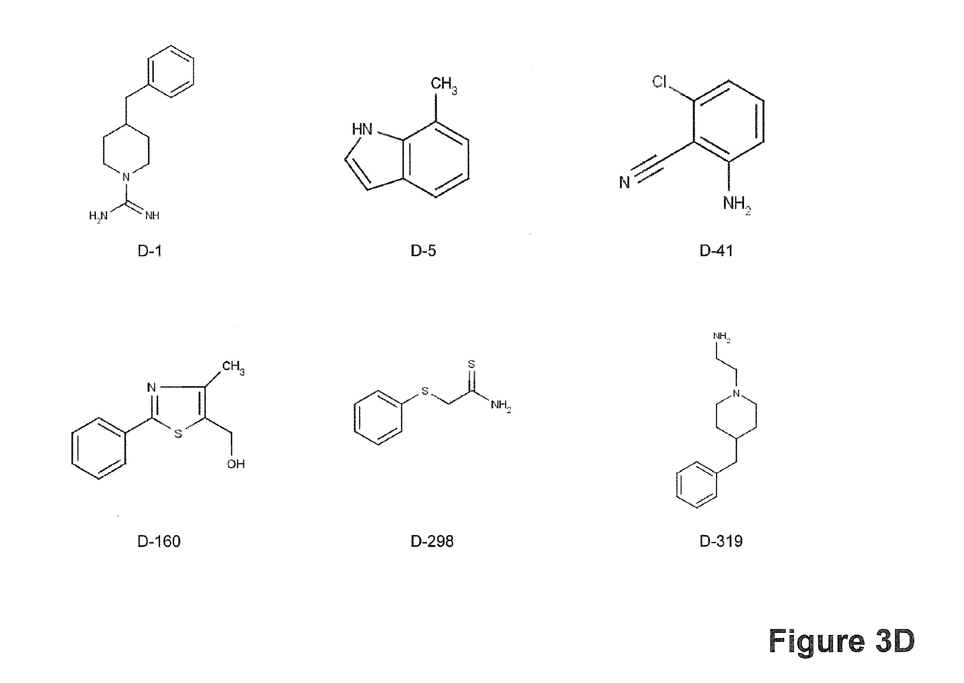

The above compounds stabilize conformational state 1 of Ras GTP by binding to one of regions A, B and C on the surface of Ras in state 1(T) (see **Figure 1).** The structural formulae of the compounds (Ras GTP binding moieties) according to the present invention are depicted in **Figure 3**.

In one embodiment, said anti-tumor agent is used for the treatment of cancer associated with oncogenic mutations of Ras.

In one embodiment, said cancer associated with oncogenic mutations of Ras is selected from the group consisting of adenocarcinomas of the pancreas, colon and lung, seminoma, thyroid tumors and myeloid leukemia.

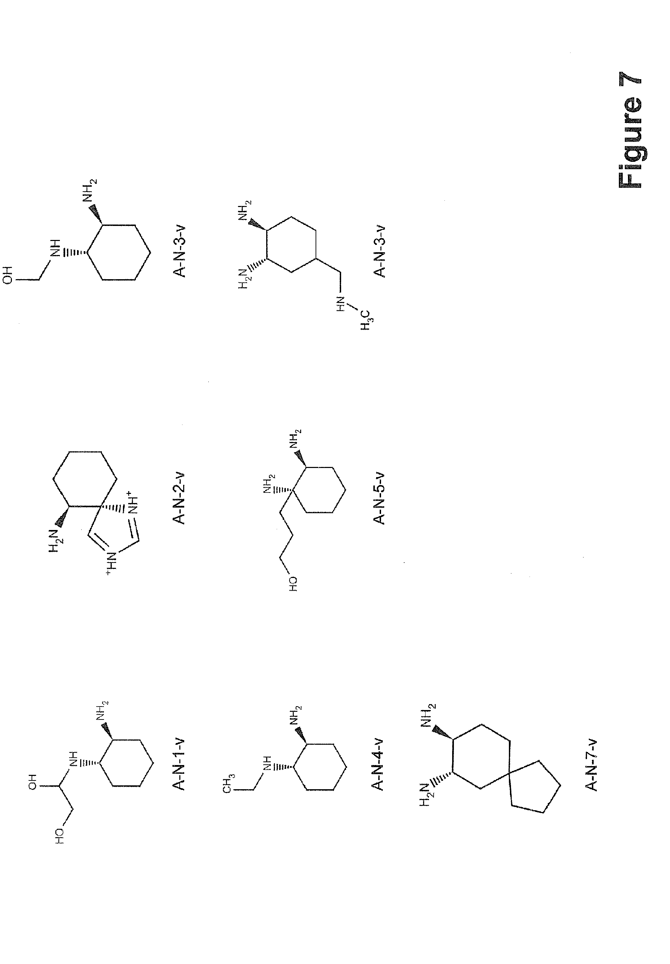

In one embodiment, said compound is (1S,2S)-cyclohexane-1,2-diamine and said derivatives are selected from the group consisting of 1-((1S,2S)-2-aminocyclohexylamino)ethane-1,2-diol, (5S,6S)-6-amino-1,3-diazoniaspiro[4.5]deca-1,3-diene, ((1S,2S)-2-aminocyclohexylamino)methanol, (1S,2S)-N1-ethylcyclohexane-1,2-diamine, 3-((1R,2S)-1,2-diaminocyclohexyl)propan-1-ol, (1S,2S)-4-((methylamino)methyl)cyclohexane-1,2-diamine and (7S,8S)-spiro[4.5]decane-7,8-diamine (see **Figure 7).**

The objects of the present invention are also solved by a Ras inhibitor comprising at least two Ras GTP binding moieties, said at least two Ras GTP binding moieties being selected from a first group of compounds consisting of (1S, 2S)-cyclohexane-1,2-diamine, 3-phenyl-1H-pyrazole, (4-(1H-imidazol-1-yl)phenyl)methanol, N-methyl-1-(4-methyl-2-phenyl-1,3-thiazol-5-yl)methanamine, (4-(pyridin-4-yl)phenyl)methanol, (3-(pyridin-4-yl)phenyl)methanol, (4H-thieno[3,2-b]pyrrol-5-yl)-methanol, (2-phenoxyphenyl)methanol, benzene-1,2-diamine, 4-phenylpiperazine-1-carboximidamide, (E)-3-(2-(3,4-dihydronaphthalen-1 (2H)-ylidene)hydrazinyl)benzoic acid, N-carbamimidoyl-3,4-dihydroquinoline-1 (2H)-carboximidamide and derivatives, enantiomers, diastereomers and pharmaceutically acceptable salts of any of the foregoing; and/or

a second group of compounds consisting of (3-(1H-pyrrol-1-yl)phenyl)methanamine, 1-(imidazo[1,2-a]pyridin-6-yl)-N-methylmethanamine, N-methyl-1-(1-methyl-3-phenyl-1H-pyrazol-5-yl)methanamine, 5-chloro-2-(methylthio)aniline, 1,3-diphenylthiourea, N-(2-(phenylamino)phenyl)acetamide, N2-methyl-N2-phenylpyridine-2,5-diamine, 5-(2-cyclopentylidenehydrazinyl)-4H-1,2,4-triazole-3-thiol and derivatives, enantiomers, diastereomers and pharmaceutically acceptable salts of any of the foregoing; and/or

a third group of compounds consisting of 2,3-diamino-l-benzyl-1H-benzo[d]imidazol-3-ium, (E)-4-(benzylideneamino)phenol, 3-chloro-N-phenylaniline, 1-(2-chlorophenyl)-3-phenylthiourea, 2-(furan-2-ylmethyl)cyclopentanamine and derivatives, enantiomers, diastereomers and pharmaceutically acceptable salts of any of the foregoing; and/or

a fourth group of compounds consisting of 4-benzylpiperidine-1-carboximidamide, 7-methyl-1H-indole, 2-amino-6-chlorobenzonitrile, (4-methyl-2-phenyl-1,3-thiazol-5-yl)methanol, 2-(phenylthio)ethanethioamide, 2-(4-benzylpiperidin-1-yl)ethanamine and derivatives, enantiomers, diastereomers and pharmaceutically acceptable salts of any of the foregoing;

with the proviso that each of said at least two Ras GTP binding moieties is from a different group, wherein said at least two Ras GTP binding moieties are covalently linked via a linker moiety.

The term "Ras inhibitor comprising at least two Ras GTP binding moieties, said at least two Ras GTP binding moieties being selected from a first group of compounds, a second group of compounds, etc., wherein said at least two Ras GTP binding moieties are covalently linked via a linker moiety" is meant to refer to a scenario wherein two compounds are chosen from the respective groups and are covalently linked to each other and thus make up the Ras inhibitor. The respective compounds act as the Ras GTP binding moieties within the Ras inhibitor. It is clear to someone skilled in the art that these compounds in their non-linked form are different from the form in which they appear as Ras GTP binding moieties within the Ras inhibitor, due to the covalent linkage between them. Such covalent linkage in its simplest form may be a covalent single bond.

A person skilled in the art understands that the covalent linkage between said at least two Ras GTP binding moieties necessitates a modification of each of said at least two Ras GTP binding moieties, i.e. (at least) one functional group of each of said at least two Ras GTP binding moieties is involved in the formation of the covalent linkage.

The four groups of compounds are defined by characteristic interacting regions on the surface of Ras in state 1(T) (see **Figure 1**). Binding of ligands to site/region A, i.e. of the compounds of the first group (or group A), involves at least one of amino acids Glu62, Glu63, Ala59, Thr58, Ala35, and Tyr32 of Ras or the $\gamma$-phosphate group of GTP. Binding to site/region B, i.e. binding of the compounds of the second group (or group B), involves at least one of amino acids Phe28, Val29 and Asp30. Binding to site/region C, i.e. binding of the compounds of the third group (or group C), involves at least one of amino acids Asn85, Lys117 and Leu120. Binding to site/region D, i.e. binding of the compounds of the fourth group (or group D), involves at least one of amino acids Asp33, Ile36, Glu37, Asp38, Ser39 and Tyr40. The corresponding residues are conserved in H-, N- and K-ras.

In the Ras inhibitor according to the present invention, at least one Ras GTP binding moiety is derived from compounds of the first, second and third group (i.e. groups A, B and C) that stabilize conformational state 1 of Ras GTP. Preferably, the Ras inhibitor according to the present invention comprises one compound of the first group (i.e. group A). Due to the presence of at least one other Ras GTP binding moiety from a different group, the overall binding affinity of the Ras inhibitor to Ras GTP is significantly increased (by additive or even cooperative action of the different binding sites).

In one embodiment, said linker moiety is a water-soluble linker moiety.

In one embodiment, said water-soluble linking moiety is an oligomer or polymer, which is preferably selected from the group consisting of (poly)alkylene oxides, such as (poly)ethylene glycol, oxyamines, peptides, and nucleic acids, such as DNA (Chung et al., 2009; Hadjuk and Greer, 2007). The length of the linker is determined directly from 3D-structures of the complexes with the compounds according to the present invention. Linkers are selected according to the minimum length that is sterically and energetically possible according to a minimum of freely rotatable single bonds.

The objects of the present invention are also solved by a Ras inhibitor as defined above for use as a medicament.

The objects of the present invention are also solved by a Ras inhibitor as defined above for use as an anti-tumor agent.

In one embodiment, said anti-tumor agent is used for the treatment of cancer associated with oncogenic mutations of Ras, wherein, preferably, said cancer associated with oncogenic mutations of Ras is selected from the group consisting of adenocarcinomas of the pancreas, colon and lung, seminoma, thyroid tumors and myeloid leukemia.

The objects of the present invention are also solved by a method for producing a Ras inhibitor as defined above, said method comprising the steps of:

- providing at least two Ras GTP binding moieties as defined above; and
- covalently linking said at least two Ras GTP binding moieties by means of a linker moiety as defined above.

The objects of the present invention are also solved by a pharmaceutical composition comprising at least one compound as defined above or at least one Ras inhibitor as defined above.

In one embodiment, said pharmaceutical composition comprises at least two different compounds as defined above or at least two different Ras inhibitors as defined above.

In one embodiment, said pharmaceutical composition further comprises at least one of a pharmaceutically acceptable carrier, diluent and excipient.

In one embodiment, said pharmaceutical composition comprises an additional anti-tumor agent. Preferably, said additional anti-tumor agent is different from the compounds and the Ras inhibitors according to the present invention.

In one embodiment, said additional anti-tumor agent is a cell-, tissue- and/or organ-specific anti-tumor agent. In one embodiment, said cell-, tissue- and/or organ-specific anti-tumor agent is suitable for the treatment of adenocarcinomas of the pancreas, colon and lung, seminoma, thyroid tumors and myeloid leukemia.

The objects of the present invention are also solved by the use of a compound as defined above or of a Ras inhibitor as defined above for inhibiting Ras *in vitro.*

The objects of the present invention are also solved by the use of a compound as defined above or a Ras inhibitor as defined above for the manufacture of a medicament for the treatment of cancer associated with oncogenic mutations of Ras.

The objects of the present invention are also solved by a method of treatment of cancer associated with oncogenic mutations of Ras, said method comprising the administration of an effective amount of a compound as defined above or a Ras inhibitor as defined above or a pharmaceutical composition as defined above to a person in need thereof.

In one embodiment, said cancer associated with oncogenic mutations of Ras is selected from the group consisting of adenocarcinomas of the pancreas, colon and lung, seminoma, thyroid tumors and myeloid leukemia.

The term "Ras", as used herein, refers to any of the mammalian, preferably human, proteins H-Ras, K-Ras and N-Ras as well as to their oncogenic mutants.

The term "(conformational) state 1 of Ras GTP", as used herein, refers to the conformational state 1(T) with low affinity to effectors (Geyer et al., 1996; Kalbitzer et al., 2009; Spoemer et al., 2001, 2005a, 2010; Rosnizeck et al., 2010). The different conformational states of Ras GTP are known to a person skilled in the art and can be distinguished, for example, by different chemical shift values in $^{31}$P NMR spectroscopy using, as a probe, the nucleotide which is bound in the active center.

FIGURES

**Figure 1** shows the positions used for virtual screening. Position A corresponds to a region centered at the $\gamma$-phosphate group of the nucleotide with a radius of 0.6 nm. For position B, the GNP-O2' was taken as centre with a radius of 0.5 nm. For position C, GNP-N7 was taken as center and a screening-radius of 0.7 nm was chosen. Position D is located outside the active site. The center of screening is the nitrogen atom of isoleucin 36 (Ile36-N), with a radius of 0.45 nm, which would allow direct linkage to compounds recognizing position A. The different areas are only depicted schematically, as there are actually no gaps between these areas.

**Figure 2** shows the reactive groups excluded in the virtual screening.

**Figure 3** shows the compounds (Ras GTP binding moieties) according to the present invention (binding in region A, B, C or D), wherein

substance A-9 is 3-phenyl-1H-pyrazole;

substance A-135 is (4-(1H-imidazol-1-yl)phenyl)methanol;

substance A-161 is N-methyl-1-(4-methyl-2-phenyl-1,3-thiazol-5-yl)methanamine;

substance A-176 is (4-(pyridin-4-yl)phenyl)methanol;

substance A-177 is (3-(pyridin-4-yl)phenyl)methanol;

substance A-192 is (4H-thieno[3,2-b]pyrrol-5-yl)-methanol;

substance A-232 is 2-phenoxyphenyl)methanol;

substance A-N is (1S,2S)-cyclohexane-1,2-diamine;

substance A-L is benzene-1,2-diamine;

substance A-B1 is 4-phenylpiperazine-1-carboximidamide;

substance A-B2 is (E)-3-(2-(3,4-dihydronaphthalen-1(2H)ylidene)hydrazinyl)benzoic acid;

substance A-B4 is N-carbamimidoyl-3,4-dihydroquinoline-1(2H)-carboximidamide.

substance B-143 is (3-(1H-pyrrol-1-yl)phenyl)methanamine;

substance B-169 is 1-(imidazo[1,2-a]pyridin-6-yl)-N-methylmethanamine;

substance B-217 is N-methyl-1-(1-methyl-3-phenyl-1H-pyrazol-5-yl)methanamine;

substance B-254 is 5-chloro-2-(methylthio)aniline;

substance B-P3-15-v is 1,3-diphenylthiourea;

substance B-P3Z-2-v is N-(2-(phenylamino)phenyl)acetamide;

substance B-P3Z-5-v is N2-methyl-N2-phenylpyridine-2,5-diamine;

substance B-P3Z-12-v is 5-(2-cyclopentylidenehydrazinyl)-4H-1,2,4-triazole-3-thiol;

substance C-P4Z-4-v is 2,3-diamino-1-benzyl-1H-benzo[d]imidazol-3-ium;

substance C-P4Z-8-v is (E)-4-(benzylideneamino)phenol;

substance C-P4Z-12-v is 3-chloro-N-phenylaniline;

substance C-P4-6-v is 1-(2-chlorophenyl)-3-phenylthiourea;

substance C-P4-10-v is 2-(furan-2-ylmethyl)cyclopentanamine;

substance D-1 is 4-benzylpiperidine-1-carboximidamide;

substance D-5 is 7-methyl-1H-indole;

substance D-41 is 2-amino-6-chlorobenzonitrile;

substance D-160 is (4-methyl-2-phenyl-1,3-thiazol-5-yl)methanol;

substance D-298 is 2-(phenylthio)ethanethioamide; and

substance D-319 is 2-(4-benzylpiperidin-1-yl)ethanamine.

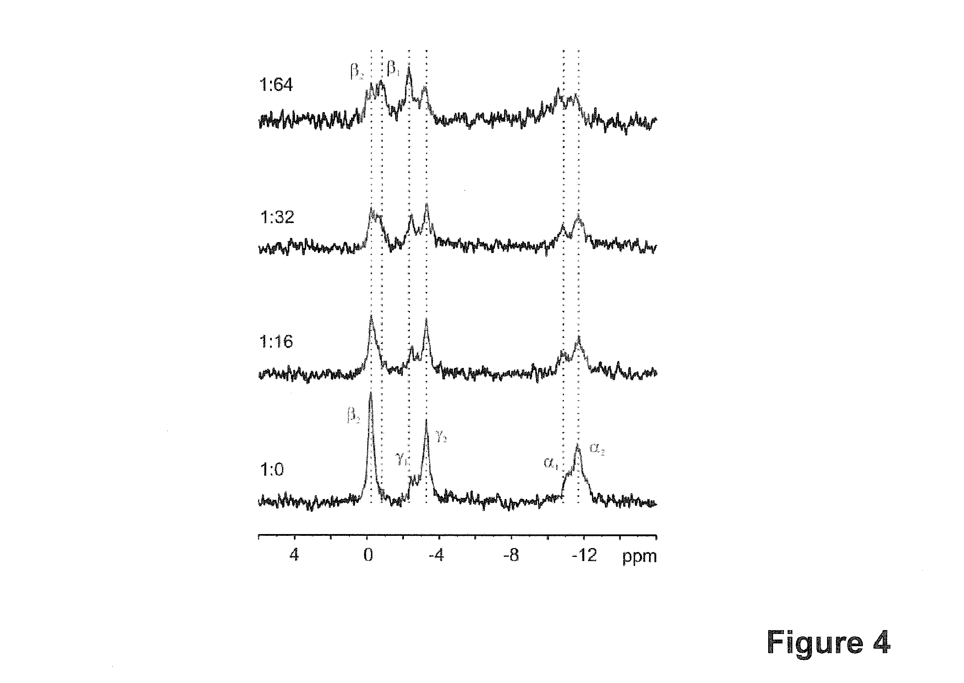

**Figure 4** shows $^{31}$P-NMR detection of the ligand-induced shift of the equilibrium towards state 1(T). (1S,2S)-cyclohexane-1,2-diamine (Substance A-N) was added to a solution of 1.5 mM Ras(wt).Mg$^{2+}$-GppNHp in 40 mM Tris/HC1, pH=7.4, 10 mM MgCl$_2$, 0.2 mM DSS and 10% D$_2$O. The temperature was 278 K. The population of state 1 detected by the phosphorus signals of GppNHp increases after addition of substance A-N.

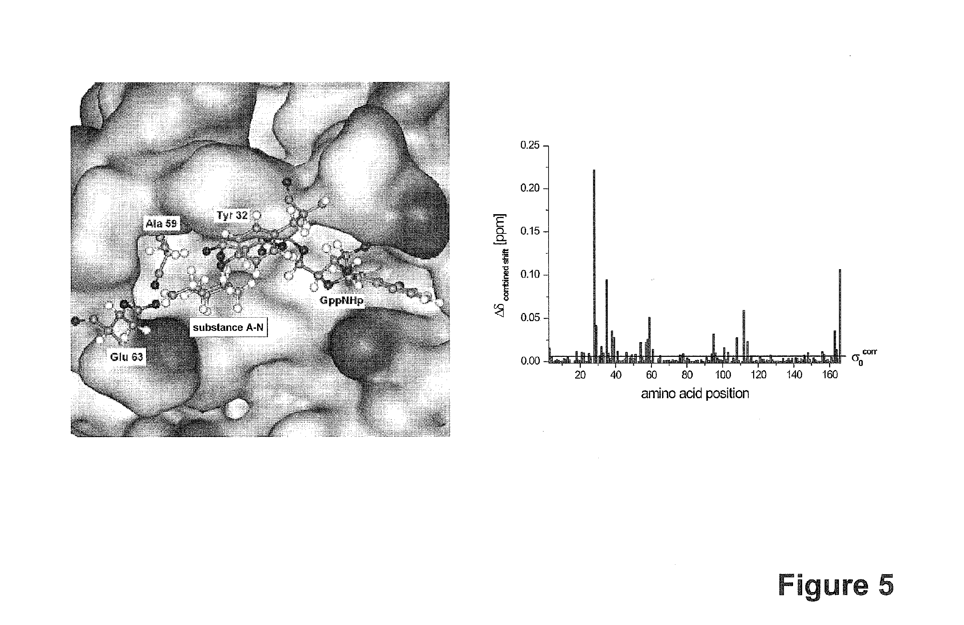

**Figure 5** shows the binding site of substance A-N as proposed by LUDI (left panel) and the chemical shift perturbation $\Delta\delta_{comb}$ obtained by interaction of substance A-N with Ras(T35A)·Mg$^{2+}$.GppNHp. The sample contained 0.1 mM $^{15}$N-enriched Ras(T35A)·Mg$^{2+}$·GppNHp. The broken line represents $\sigma_0$ calculated according to Schumann et al. (2007).

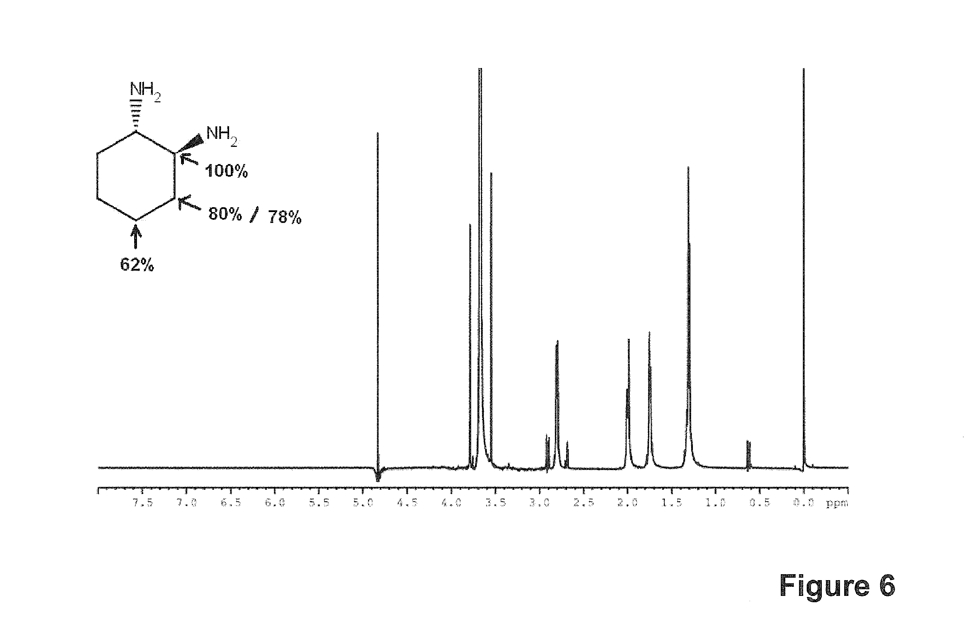

**Figure 6** shows the STD spectrum (left panel) and the amplification factors (right panel) for substance A-N. For the proton in position 5 and 6, the amplification factor is close to zero.

**Figure 7** shows exemplary derivatives of substance A-N.

EXAMPLES

The inventors have previously shown that stabilization of state 1(T) by Zn$^{2+}$-cyclen reduces the affinity of Ras for effectors significantly. However, the affinity of this compound was much too low for practical applications. Therefore, the inventors started a systematic approach to obtain high affinity state 1(T) inhibitors using the following strategy: (1) virtual screening by LUDI of the state 1(T) structure determined by NMR (Rosnizeck et al., 2010) with a small compound library consisting of 9.74 million small compounds; (2) experimental NMR-based high-throughput screening (including determination of the corresponding dissociation constants) of promising candidates including candidates from virtual screening as well as candidates from experimental data bases (e.g. the Maybridge library); (3) identification of the binding site on the protein surface by chemical shift perturbation; and (4) creation of high affinity compounds on the basis of the available experimental data by addition of appropriate substituents to the lead structure and/or by linking of the small compounds that have non-overlapping binding sites in close neighbourhood or by modifying them to obtain high affinity binders.

Virtual Screening using LUDI

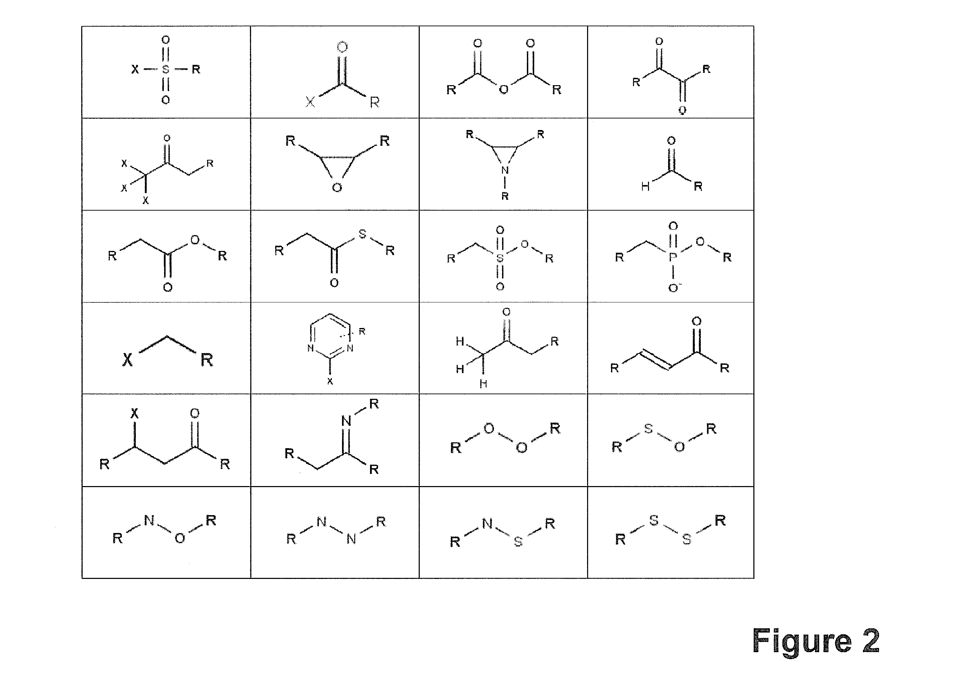

First, a virtual database of 9.6 million compounds was prepared that contained small compounds fulfilling modified "drug-likeness" conditions (MW, molecular weight; Rings, number of aromatic rings; RTB, freely rotatable bonds, HAc, hydrogen bond acceptors; HDo, hydrogen bond donors; logP, hydrophobicity) based on Lipinski's Rule-of-Five and the exclusion of reactive groups (Oprea, 2000) (see **Table 1** and **Figure 2).**

Table 1. Modified Rule-of-Five criteria.

| $0 \leq$ | MW | $\leq 500$ |
|---|---|---|
| $2 \leq$ | RTB | $\leq 15$ |
| $-3.5 \leq$ | logP | $\leq 4.5$ |
| $2 \leq$ | HAc | $\leq 9$ |
| $0 \leq$ | HDo | $\leq 5$ |
| $1 \leq$ | rings | $\leq 4$ |

These compounds were screened with the program LUDI against the state 1 structure of Ras·$Mg^{2+}$·GppNHp as determined by the inventors in the presence of $Zn^{2+}$-cyclen (Graf, 2006; Rosnizeck et al., 2010). Four different positions were selected (see **Figure 1).** Position A, B, and C are only accessible in state 1 — binding to these positions therefore should stabilize this state. Binding to position D is possible in the two states and may mainly be used in the linked-fragment approach for enhancing the affinity of the Ras inhibitor.

Experimental confirmation of Ras-binding

Selected compounds identified by virtual screening were further studied by NMR spectroscopy. The interaction of the ligands with Ras was determined experimentally by different methods using the mutant Ras(T35A)·$Mg^{2+}$·GppNHp as model for state 1. Saturation transfer difference (STD) spectroscopy (Mayer and Meyer, 1999, 2001) or Water-LOGSY (Dalvit et al., 2001) were used to prove binding of the selected ligand with the protein. In case binding was detectable, the dissociation constant $K_D$ was determined by measurements at different ligand concentrations. Alternatively, the $K_D$ as well as the binding site of the ligand was determined by using uniformly $^{15}$N-enriched Ras(T35A) using $^1$H,$^{15}$N-HSQC spectroscopy (Palmer et al. 1991; Shaka et al. 1985; Schleucher et al. 1994) or $^1$H,$^{15}$N-sofast HMQC spectroscopy (Schanda and Brutscher, 2005; Schanda et al., 2005). The $K_D$ was determined by fitting the amino acid-specific combined chemical shift $\delta_{comb}$ (Schumann et al., 2007) of well-resolved amide resonances as a function of the total concentration $c_1$ of the ligand according to the formula

$$\delta_{comb} = (\delta_{comb}^{c} - \delta_{comb}^{f}) \frac{c_p^0 + c_l^0 + K_D - \sqrt{(c_p^0 + c_l^0 + K_D)^2 - 4c_p^0 c_l^0}}{2c_p^0} + \delta_{comb}^{f}$$

(see **Figure 5).**

Here, $\delta_{comb}^{f}$ and $\delta_{comb}^{c}$ are the chemical shifts in the free and the completely saturated state of the protein, $c_p$ is the protein concentration. For the evaluation of STD measurements $\delta_{comb} - \delta_{comb}^{f}$ has to be replaced by the amplification factor $(I-I_0)/I_0$ and $\delta_{comb}^{c} - \delta_{comb}^{f}$ represents a fit parameter. In cases, where the dissociation constant was much higher than the used protein concentration (as it is usually the case in STD-measurements), a simplified relation can be used (Mayer and Meyer, 1999). By using the above approach, 22 compounds could be identified with dissociation constants in the range of 10 micromolar to millimolar.

Identification of state 1 (T) inhibitors

Although it can be assumed that ligands that preferentially bind to positions A, B, or C defined as above are state 1(T) inhibitors, this property can be further checked by $^{31}$P NMR spectroscopy on wildtype Ras·Mg$^{2+}$·GppNHp, since a shift from state 2 to state 1 can be observed directly in the NMR spectra. Several small compounds that stabilize state 1(T) by binding selectively to this state were found. An example is shown in **Figure 4** where substance A-N (i.e. (1S,2S)-cyclohexane-1,2-diamine) is added to a solution that contains wildtype Ras in complex with Mg$^{2+}$·GppNHp. The relative population of state 1 and state 2 can be obtained by integration of the γ-phosphate line. Addition of the small ligand A-N leads to a clear stabilization for the weak binding state for effectors (state 1).

The chemical shift perturbation $\Delta\delta_{comb}$ obtained by SOFAST-HMQC-spectroscopy delineates the binding site of substance A-N. Amino acids mainly perturbed are Phe28, Val29, Ala35, Asp38, and Ala59. They are in good agreement with the result from LUDI **(Figure 5)**. It predicts hydrogen bonds of the amino groups with the γ-phosphate of the nucleotide, the carbonyl group of Ala59, and the carboxyl group of Glu63. In case that a more detailed binding pattern is desired more elaborated docking programs such as HADDOCK should be used (Dominguez et al., 2003) which allow small structural rearrangements in the binding site.

Creation of compounds with higher affinity

There are two different strategies to obtain compounds with even higher affinity from the experimental data presented herein that can be followed also in combination: the lead  structures found (e.g. substance A-N) can be modified by addition or removal of small functional groups and/or linked to lead structures binding in the neighborhood, i.e. regions B, C, and/or D. Programs such as LUDI can be used for the proposition of modification and linkers. Besides the structural information, an additional feature produced in the data set can be used to find the appropriate regions for modification in the lead structure: atoms showing a low amplification factor are most probably not in direct contact with the target protein and are preferred positions for a modification (see **Figure 6)**. Examples for such modifications of substance A-N are:

1-((1S,2S)-2-aminocyclohexylamino)ethane-1,2-diol (substance A-N-1-v);
(SS,6S)-6-amino-1,3-diazoniaspiro[4.5]deca-1,3-diene (substance A-N-2-v);
((1S,2S)-2-aminocyclohexylamino)methanol (substance A-N-3-v);
(1S,2S)-N1-ethylcyclohexane-1,2-diamine (substance A-N-4-v);
3-((1R,2S)-1,2-diaminocyclohexyl)propan-1-ol (substance A-N-5-v);
(1S,2S)-4-((methylamino)methyl)cyclohexane-1,2-diamine (substance A-N-6-v); and
(7S,8S)-spiro[4.5]decane-7,8-diamine (substance A-N-7-v).

The corresponding structures are shown in **Figure 7.**

REFERENCES

Bos, J.L. (1989) Cancer Res 49: 4682-4689.
Chung, S., et al. (2009) Nature Chem. Biol. 5, 407-413.
Dalvit, C., et al. (2001) J. Biomol. NMR 21: 349-359.
Dominguez, C., et al. (2003) J. Am. Chem. Soc. 125: 1731-1737.
Geyer, M., et al. (1996) Biochemistry 35, 10308-10320.
Graf, T. (2006) Entwicklung neuer Inhibitoren für das Ras-Protein und dessen onkogene Mutanten auf Basis von Cyclenderivaten. PhD thesis, University of Regensburg.
Hadjuk, P. J. and Greer, J. (2007) Nature Rev. 6, 211-219.
Kalbitzer H.R. and König, B. 140:122769 AN 2004:60322.
Kalbitzer H.R., et al. (2009) J. Am. Chem. Soc. 131: 16714-16719.
Mayer, M. and Meyer B. (1999) Angew. Chem. Internat. Ed. 38: 1784-1788.
Mayer, M. and Meyer B. (2001) J. Am. Chem. Soc. 123: 6108-6117.
Neumann, T., et al. (2007) Curr. Topics Med. Chem. 7: 1630-1642.
Oprea, T. I. (2000) J. Compuer-Aided Mol. Design 14: 251-264.
Palmer III, A.G., et al. (1991) J. Magn. Reson. 93: 151-170.
Rosnizeck, I.C., et al. (2010) Angew. Chem. Int. Ed. 49: 3830-3833.
Schanda, P. and Brutscher B. (2005) J Am Chem Soc 127: 8014-8015.

Schanda, P., et al. (2005) J. Biomol. NMR 33, 199-211.
Schleucher, J., et al. (1994) J. Biomol. NMR 4, 301-306.
Schmidt, F., et al. (2011) Inorg. Chim. Acta 365: 38-48.
Schumann, F.H., et al. (2007) J. Biomol. NMR 39, 275-289.
Shaka, A.J., et al. (1985) J. Magn. Reson. 64, 547-552.
Spoerner, M., et al. (2001) Proc. Natl. Acad. Sci. U.S.A. 98: 4944-4949.
Spoerner, M., et al. (2007) FEBS J. 274: 1419-1433.
Spoerner, M., et al. (2005a) Biochemistry 44: 2225-2236.
Spoerner, M., et al. (2005b) Biochem. Biophys. Res. Commun. 334: 709-713.
Spoerner, M., et al. (2010) J. Biol. Chem. 285: 39768-39778.
Wittinghofer, A. and Waldmann, H. (2000) Angew. Chem. Int. Ed. 39: 4193-4214.

**Claims**

1. Compound selected from the group consisting of (1S,2S)-cyclohexane-1,2-diamine, 3-phenyl-1H-pyrazole, (4-(1H-imidazol-1-yl)phenyl)methanol, N-methyl-1-(4-methyl-2-phenyl-1,3-thiazol-5-yl)methanamine, (4-(pyridin-4-yl)phenyl)methanol, (3-(pyridin-4-yl)phenyl)methanol, (4H-thieno[3,2-b]pyrrol-5-yl)-methanol, (2-phenoxyphenyl)methanol, benzene-1,2-diamine, 4-phenylpiperazine-1-carboximidamide, (E)-3-(2-(3,4-dihydronaphthalen-1(2H)-ylidene)hydrazinyl)benzoic acid, N-caxbamimidoyl-3,4-dihydroquinoline-1(2H)-carboximidamide, (3-(1H-pyrrol-1-yl)phenyl)methanamine; 1-(imidazo[1,2-a]pyridin-6-yl)-N-methylmethanamine; N-methyl-1-(1-methyl-3-phenyl-1H-pyrazol-5-yl)methanamine; 5-chloro-2-(methylthio)aniline; 1,3-diphenylthiourea; N-(2-(phenylamino)phenyl)acetamide; N2-methyl-N2-phenylpyridine-2,5-diamine; 5-(2-cyclopentylidenehydrazinyl)-4H-1,2,4-triazole-3-thiol, 2,3-diamino-1-benzyl-1H-benzo[d]imidazol-3-ium; (E)-4-(benzylideneamino)phenol; 3-chloro-N-phenylaniline; 1-(2-chlorophenyl)-3-phenylthiourea; 2-(furan-2-ylmethyl)cyclopentanamine and derivatives, enantiomers, diastereomers and pharmaceutically acceptable salts of any of the foregoing for use as a medicament.

2. Compound selected from the group consisting of (1S,2S)-cyclohexane-1,2-diamine, 3-phenyl-1H-pyrazole, (4-(1H-imidazol-1-yl)phenyl)methanol, N-methyl-1-(4-methyl-2-phenyl-1,3-thiazol-5-yl)methanamine, (4-(pyridin-4-yl)phenyl)methanol, (3-(pyridin-4-yl)phenyl)methanol, (4H-thieno[3,2-b]pyrrol-5-yl)-methanol, (2-phenoxyphenyl)methanol, benzene-1,2-diamine, 4-phenylpiperazine-1-carboximidamide, (E)-3-(2-(3,4-dihydronaphthalen-1 (2H)-ylidene)hydrazinyl)benzoic acid, N-carbamimidoyl-3,4-dihydroquinoline-1(2H)-carboximidamide, (3-(1H-pyrrol-1-yl)phenyl)methanamine; 1-(imidazo[1,2-a]pyridin-6-yl)-N-methylmethanamine; N-methyl-1-(1-methyl-3-phenyl-1H-pyrazol-5-yl)methanamine; 5-chloro-2-(methylthio)aniline; 1,3-diphenylthiourea; N-(2-(phenylamino)phenyl)acetamide; N2-methyl-N2-phenylpyridine-2,5-diamine; 5-(2-cyclopentylidenehydrazinyl)-4H-1,2,4-triazole-3-thiol, 2,3-diamino-1-benzyl-1H-benzo[d]imidazol-3-ium; (E)-4-(benzylideneamino)phenol; 3-chloro-N-phenylaniline; 1-(2-chlorophenyl)-3-phenylthiourea; 2-(furan-2-ylmethyl)cyclopentanamine and derivatives, enantiomers, diastereomers and pharmaceutically acceptable salts of any of the foregoing for use as an anti-tumor agent.

3. Compound according to claim 2, wherein said anti-tumor agent is used for the treatment of cancer associated with oncogenic mutations of Ras.

4. Compound according to claim 3, wherein said cancer associated with oncogenic mutations of Ras is selected from the group consisting of adenocarcinomas of the pancreas, colon and lung, thyroid tumors and myeloid leukemia.

5. Compound according to any of the foregoing claims, wherein said compound is (1S,2S)-cyclohexane-1,2-diamine and said derivatives are selected from the group consisting of 1-((1S,2S)-2-aminocyclohexylamino)ethane-1,2-diol, (5S,6S)-6-amino-1,3-diazoniaspiro[4.5]deca-1,3-diene, ((1S,2S)-2-aminocyclohexylamino)methanol, (1S,2S)-N1-ethylcyclohexane-1,2-diamine, 3-((1R,2S)-1,2-diaminocyclohexyl)propan-1-ol, (1S,2S)-4-((methylamino)methyl)cyclohexane-1,2-diamine and (7S,8S)-spiro[4.5]decane-7,8-diamine.

6. Ras inhibitor comprising at least two Ras GTP binding moieties, said at least two Ras GTP binding moieties being selected from
a first group of compounds consisting of (1S,2S)-cyclohexane-1,2-diamine, 3-phenyl-1 H-pyrazole, (4-(1H-imidazol-1-yl)phenyl)methanol, N-methyl-1-(4-methyl-2-phenyl-1,3-thiazol-5-yl)methanamine, (4-(pyridin-4-yl)phenyl)methanol, (3-(pyridin-4-yl)phenyl)methanol, (4H-thieno[3,2-b]pyrrol-5-yl)-methanol, (2-phenoxyphenyl)methanol, benzene-1,2-diamine, 4-phenylpiperazine-1-carboximidamide, (E)-3-(2-(3,4-dihydronaphthalen-1(2H)-ylidene)hydrazinyl)benzoic acid, N-carbamimidoyl-3,4-dihydroquinoline-1(2H)-carboximidamide and derivatives, enantiomers, diastereomers and pharmaceutically acceptable salts of any of the foregoing; and/or

a second group of compounds consisting of (3-(1H-pyrrol-1-yl)phenyl)methanamine, 1-(imidazo[1,2-a]pyridin-6-yl)-N-methylmethanamine, N-methyl-1-(1-methyl-3-phenyl-1H-pyrazol-5-yl)methanamine, 5-chloro-2-(methylthio) aniline, 1,3-diphenylthiourea, N-(2-(phenylamino)phenyl)acetamide, N2-methyl-N2-phenylpyridine-2,5-diamine, 5-(2-cyclopentylidenehydrazinyl)-4H-1,2,4-triazole-3-thiol and derivatives, enantiomers, diastereomers and pharmaceutically acceptable salts of any of the foregoing; and/or

a third group of compounds consisting of 2,3-diamino-1-benzyl-1H-benzo[d]imidazol-3-ium, (E)-4-(benzylideneamino)phenol, 3-chloro-N-phenylaniline, 1-(2-chlorophenyl)-3-phenylthiourea, 2-(furan-2-ylmethyl)cyclopentanamine and derivatives, enantiomers, diastereomers and pharmaceutically acceptable salts of any of the foregoing; and/or

a fourth group of compounds consisting of 4-benzylpiperidine-1-carboximidamide, 7-methyl-1H-indole, 2-amino-6-chlorobenzonitrile, (4-methyl-2-phenyl-1,3-thiazol-5-yl)methanol, 2-(phenylthio)ethanethioamide, 2-(4-benzylpiperidin-1-yl)ethanamine and derivatives, enantiomers, diastereomers and pharmaceutically acceptable salts of any of the foregoing;

with the proviso that each of said at least two Ras GTP binding moieties is from a different group,

wherein said at least two Ras GTP binding moieties are covalently linked via a linker moiety.

7. Ras inhibitor according to claim 6, wherein said linker moiety is a water-soluble linker moiety.

8. Ras inhibitor according to claim 7, wherein said water-soluble linker moiety is selected from the group consisting of (poly)alkylene oxides, such as (poly)ethylene glycol, oxyamines, peptides and nucleic acids, such as DNA.

9. Ras inhibitor according to any of claims 6 to 8 for use as a medicament.

10. Ras inhibitor according to any of claims 6 to 8 for use as an anti-tumor agent.

11. Ras inhibitor according to claim 10, wherein said anti-tumor agent is used for the treatment of cancer associated with oncogenic mutations of Ras, wherein, preferably, said cancer associated with oncogenic mutations of Ras is selected from the group consisting of adenocarcinomas of the pancreas, colon and lung, thyroid tumors, seminoma, and myeloid leukemia.

12. Pharmaceutical composition comprising at least one compound according to any of claims 1 to 5 or at least one Ras inhibitor according to any of claims 6 to 11.

13. Pharmaceutical composition according to claim 12, comprising an additional anti-tumor agent.

14. Pharmaceutical composition according to claim 13, wherein said additional anti-tumor agent is a cell-, tissue- and/or organ-specific anti-tumor agent.

15. Use of a compound as defined in any of claims 1 to 5 or of a Ras inhibitor as defined in any of claims 6 to 8 for inhibiting Ras *in vitro.*

Figure 1

**Figure 2**

A-9           A-135          A-161          A-176

A-177          A-192          A-232          A-N

A-L           A-B1           A-B2           A-B4

**Figure 3A**

EP 2 671 575 A1

B-143　　　　　B-169　　　　　B-217　　　　　B-254

B-P3Z-5-v　　　　B-P3Z-12-v　　　　B-P3Z-2-v　　　　B-P3-15-v

Figure 3B

EP 2 671 575 A1

13

Figure 3C

D-41

D-319

D-5

D-298

D-1

D-160

Figure 3D

Figure 4

Figure 5

Figure 6

Figure 7

Europäisches
Patentamt

European
Patent Office

Office européen
des brevets

# EUROPEAN SEARCH REPORT

**Application Number**

EP 12 17 0715

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PT 1 442 050 E (MALESCI SAS [IT]) 31 January 2007 (2007-01-31) * See the diamino cyclohexyl compounds described in claim 3, listed from page 4/18, line 20 to page 6/18, line 7 and their use in the treatment of cancer (page 64, lines 19-20 and claim 9, (page 18/18, line 7) * | 1,2,5,12 | INV. A61K31/132 A61K35/02 |
| X | EP 0 264 109 A1 (TORAY INDUSTRIES [JP]) 20 April 1988 (1988-04-20) * See the 1S,2S diaminocyclohexyl compounds disclosed in examples 1-33 and their use for the treatment of cancer: (page 3, lines 7, 24-25). * | 1,2,5,12 | |
| X | WO 89/04319 A1 (TORAY INDUSTRIES [JP]) 18 May 1989 (1989-05-18) * See the 1S,2S diaminocyclohexyl compounds of claim 3 and their use in the treatment of cancer * | 1,2,5,12 | |
| X | WO 2004/089962 A2 (YISSUM RES DEV CO [IL]; BREUER ELI [IL]; REICH REUVEN [IL]; GOLOMB GER) 21 October 2004 (2004-10-21) * See the 1S 2S cyclohexylderivative 11a at page 12 and its use for the treatment of cancers (claims 15, 17, 19) * | 1,2,12 | TECHNICAL FIELDS SEARCHED (IPC) A61K |
| X | EP 2 206 506 A1 (BRACCO IMAGING SPA [IT]) 14 July 2010 (2010-07-14) * See trans-1,2 diaminocyclohexane and pharmaceutical compositions thereof * | 1,12 | |

-/--

~~The present search report has been drawn up for all claims~~

| Place of search | Date of completion of the search | Examiner |
|---|---|---|
| Munich | 6 September 2012 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 12 17 0715

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/029054 A1 (UNIV MICHIGAN [US]; UNIV VIRGINIA [US]; HESS JAY [US]; GREMBECKA JOLAN) 10 March 2011 (2011-03-10) * See pages 38-40 and claim 13; compound N. 45 (4A6), as inhibitor of the menin-MLL oncoprotein interaction; pharmaceutical compositions thereof. See page 2, use of such inhibitors for the treatment of leukemia * | 1,2,5,12 | |
| X | WO 2010/138820 A2 (HARVARD COLLEGE [US]; AKTAS HUSEYIN [US]; HALPERIN JOSE A [US]; CHOREV) 2 December 2010 (2010-12-02) * See claim 1 and the compound Code N.1831 at page 100 and their use in the treatment of cancer, including lung and colon cancers, adenocarcinoma and myeloid leukemia (see paragraphs 23, 25, 26, 29-32 and the figures cited therein and paragraphs 68-71). See the combinations described in paragraph 86 * | 1-4, 12-14 | |
| | | | TECHNICAL FIELDS SEARCHED (IPC) |
| X | WO 2009/073620 A2 (NEWLINK GENETICS [US]; MAUTINO MARIO [US]; JAIPURI FIROZ [US]; MARCINO) 11 June 2009 (2009-06-11) * See page 231, compound N. 1888 and its use for the treatment of cancer (page 5, claims 1, 26, 49, 54, 179). See the combinations described in paragraphs 635-640 * | 1-4, 12-14 | |

-/--

~~The present search report has been drawn up for all claims~~

| Place of search | Date of completion of the search | Examiner |
|---|---|---|
| Munich | 6 September 2012 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**EUROPEAN SEARCH REPORT**

Application Number

EP 12 17 0715

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | ROSNIZECK, I.C. ET AL.: ANGEW. CHEM. INT. ED., vol. 49, 2010, pages 3830-3833, XP002682934, * See discussion in page 3830 - 3832, figures and conclusions in page 3833: Cyclen complexes bind to two separate sites of Ras and stabilise the inactive conformational state 1, decreasing effectors affinity. * | 1-15 | |
| X,D | SPOERNER, M. ET AL.: BIOCHEM. BIOPHYS. RES. COMMUN., vol. 334, 2005, pages 709-713, XP027229720, * See abstract, discussion and discussion in pages 709-712: Zn cyclen binds to and stabilise the weak binding conformational state of Ras. * | 1-15 | |
| X,D | WO 2004/006934 A2 (KALBITZER HANS ROBERT [DE]; KOENIG BURKHARD [DE]) 22 January 2004 (2004-01-22) * See description and claims: Zn cyclen binds to and stabilise the weak binding conformational state of Ras. Use as antitumoral agent * | 1-15 | TECHNICAL FIELDS SEARCHED (IPC) |
| X,D | SCHMIDT, F. ET AL.: INORG. CHIM. ACTA, vol. 365, 2011, pages 38-48, XP027571644, * Bidentate ligands intended for binding and stabilising the weak effector binding site of Ras * | 1-15 | |

~~The present search report has been drawn up for all claims~~

| Place of search | Date of completion of the search | Examiner |
|---|---|---|
| Munich | 6 September 2012 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

..............................................................................
& : member of the same patent family, corresponding document

EPO FORM 1503 03.82 (P04C01)

Europäisches
Patentamt

European
Patent Office

Office européen
des brevets

**Application Number**

EP 12 17 0715

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

5(completely); 1-4, 6-15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 5(completely); 1-4, 6-15(partially)

     Compound (1S,2S)-cyclohexane-1,2-diamine and derivatives, in particular as defined in claims 5 and 7 and enantiomers, diastereomers and pharmaceutically acceptable salts of any of the foregoing and pharmaceutical compositions thereof, for use as a medicament, in particular for the treatment of cancer,

                     ---

2. claims: 1-4, 6-15(all partially)

     3-phenyl-1H-pyrazole and derivatives, in particular as defined in claim 7 and enantiomers, diastereomers and pharmaceutically acceptable salts of any of the foregoing and pharmaceutical compositions thereof, for use as a medicament, in particular for the treatment of cancer,

                     ---

3. claims: 1-4, 6-15(all partially)

     (4-(1H-imidazol-1-yl)phenyl)methanol and derivatives, in particular as defined in claim 7 and enantiomers, diastereomers and pharmaceutically acceptable salts of any of the foregoing and pharmaceutical compositions thereof, for use as a medicament, in particular for the treatment of cancer,

                     ---

4. claims: 1-4, 6-15(all partially)

     N-methyl-1-(4-methyl-2-phenyl-1,3-thiazol-5-yl)methanamine and derivatives, in particular as defined in claim 7 and enantiomers, diastereomers and pharmaceutically acceptable salts of any of the foregoing and pharmaceutical compositions thereof, for use as a medicament, in particular for the treatment of cancer,

                     ---

5. claims: 1-4, 6-15(all partially)

     (4-(pyridin-4-yl)phenyl)methanol and derivatives, in particular as defined in claim 7 and enantiomers, diastereomers and pharmaceutically acceptable salts of any of the foregoing and pharmaceutical compositions thereof, for use as a medicament, in particular for the treatment of cancer,

                     ---

6. claims: 1-4, 6-15(all partially)

Europäisches
Patentamt

European
Patent Office

Office européen
des brevets

**LACK OF UNITY OF INVENTION
SHEET B**

Application Number

EP 12 17 0715

The Search Division considers that the present European patent application does not comply with the
requirements of unity of invention and relates to several inventions or groups of inventions, namely:

(3 -(pyridin-4-yl)phenyl)methanol and derivatives, in
particular as defined in claim 7 and enantiomers,
diastereomers and pharmaceutically acceptable salts of any
of the foregoing and pharmaceutical compositions thereof,
for use as a medicament, in particular for the treatment of
cancer,
---

7. claims: 1-4, 6-10(all partially)

(4H-thieno[3,2-b]pyrrol-5-yl)-methanol and derivatives, in
particular as defined in claim 7 and enantiomers,
diastereomers and pharmaceutically acceptable salts of any
of the foregoing and pharmaceutical compositions thereof,
for use as a medicament, in particular for the treatment of
cancer,
---

8. claims: 1-4, 6-15(all partially)

(2-phenoxyphenyl)methanol and derivatives, in particular as
defined in claim 7 and enantiomers, diastereomers and
pharmaceutically acceptable salts of any of the foregoing
and pharmaceutical compositions thereof, for use as a
medicament, in particular for the treatment of cancer,
---

9. claims: 1-4, 6-15(all partially)

benzene-1,2-diamine and derivatives, in particular as
defined in claim 7 and enantiomers, diastereomers and
pharmaceutically acceptable salts of any of the foregoing
and pharmaceutical compositions thereof, for use as a
medicament, in particular for the treatment of cancer,
---

10. claims: 1-4, 6-15(all partially)

4-phenylpiperazine-1-carboximidamide and derivatives, in
particular as defined in claim 7 and enantiomers,
diastereomers and pharmaceutically acceptable salts of any
of the foregoing and pharmaceutical compositions thereof,
for use as a medicament, in particular for the treatment of
cancer,
---

11. claims: 1-4, 6-15(all partially)

(E)-3-(2-(3,4-dihydronaphthalen-1(2H)-ylidene)hydrazinyl)ben
zoic acid and derivatives, in particular as defined in claim
7 and enantiomers, diastereomers and pharmaceutically

Europäisches
Patentamt

European
Patent Office

Office européen
des brevets

**LACK OF UNITY OF INVENTION
SHEET B**

Application Number

EP 12 17 0715

The Search Division considers that the present European patent application does not comply with the
requirements of unity of invention and relates to several inventions or groups of inventions, namely:

acceptable salts of any of the foregoing and pharmaceutical
compositions thereof, for use as a medicament, in particular
for the treatment of cancer,

---

12. claims: 1-4, 6-15(all partially)

N-carbamimidoyl-3,4-dihydroquinoline-1(2H)-carboximidamide
and derivatives, in particular as defined in claim 7 and
enantiomers, diastereomers and pharmaceutically acceptable
salts of any of the foregoing and pharmaceutical
compositions thereof, for use as a medicament, in particular
for the treatment of cancer,

---

13. claims: 1-4, 6-15(all partially)

(3-(1H-pyrrol-1-yl)phenyl)methanamine and derivatives, in
particular as defined in claim 7 and enantiomers,
diastereomers and pharmaceutically acceptable salts of any
of the foregoing and pharmaceutical compositions thereof,
for use as a medicament, in particular for the treatment of
cancer,

---

14. claims: 1-4, 6-15(all partially)

1-(imidazo[1,2-a]pyridin-6-yl)-N-methylmethanamine and
derivatives, in particular as defined in claim 7 and
enantiomers, diastereomers and pharmaceutically acceptable
salts of any of the foregoing and pharmaceutical
compositions thereof, for use as a medicament, in particular
for the treatment of cancer,

---

15. claims: 1-4, 6-15(all partially)

N-methyl-1-(1-methyl-3-phenyl-1H-pyrazol-5-yl)methanamine
and derivatives, in particular as defined in claim 7 and
enantiomers, diastereomers and pharmaceutically acceptable
salts of any of the foregoing and pharmaceutical
compositions thereof, for use as a medicament, in particular
for the treatment of cancer,

---

16. claims: 1-4, 6-15(all partially)

5-chloro-2-(methylthio)aniline and derivatives, in
particular as defined in claim 7 and enantiomers,
diastereomers and pharmaceutically acceptable salts of any
of the foregoing and pharmaceutical compositions thereof,

Europäisches
Patentamt

European
Patent Office

Office européen
des brevets

**LACK OF UNITY OF INVENTION
SHEET B**

Application Number

EP 12 17 0715

The Search Division considers that the present European patent application does not comply with the requirements of unity of invention and relates to several inventions or groups of inventions, namely:

for use as a medicament, in particular for the treatment of cancer,

---

17. claims: 1-4, 6-15

1,3-diphenylthiourea and derivatives, in particular as defined in claim 7 and enantiomers, diastereomers and pharmaceutically acceptable salts of any of the foregoing and pharmaceutical compositions thereof, for use as a medicament, in particular for the treatment of cancer,

---

18. claims: 1-4, 6-15(all partially)

N-(2-(phenylamino)phenyl)acetamide and derivatives, in particular as defined in claim 7 and enantiomers, diastereomers and pharmaceutically acceptable salts of any of the foregoing and pharmaceutical compositions thereof, for use as a medicament, in particular for the treatment of cancer,

---

19. claims: 1-4, 6-15(all partially)

N2-methyl-N2-phenylpyridine-2,5-diamine and derivatives, in particular as defined in claim 7 and enantiomers, diastereomers and pharmaceutically acceptable salts of any of the foregoing and pharmaceutical compositions thereof, for use as a medicament, in particular for the treatment of cancer,

---

20. claims: 1-4, 6-15(all partially)

5-(2-cyclopentylidenehydrazinyl)-4H-1,2,4-triazole-3-thiol and derivatives, in particular as defined in claim 7 and enantiomers, diastereomers and pharmaceutically acceptable salts of any of the foregoing and pharmaceutical compositions thereof, for use as a medicament, in particular for the treatment of cancer,

---

21. claims: 1-4, 6-15(all partially)

2,3-diamino-1-benzyl-1H-benzo[d]imidazol-3-ium and derivatives, in particular as defined in claim 7 and enantiomers, diastereomers and pharmaceutically acceptable salts of any of the foregoing and pharmaceutical compositions thereof, for use as a medicament, in particular for the treatment of cancer,

Europäisches
Patentamt
European
Patent Office
Office européen
des brevets

**LACK OF UNITY OF INVENTION
SHEET B**

Application Number

EP 12 17 0715

The Search Division considers that the present European patent application does not comply with the requirements of unity of invention and relates to several inventions or groups of inventions, namely:

---

22. claims: 1-4, 6-15(all partially)

(E)-4-(benzylideneamino)phenol and derivatives, in particular as defined in claim 7 and enantiomers, diastereomers and pharmaceutically acceptable salts of any of the foregoing and pharmaceutical compositions thereof, for use as a medicament, in particular for the treatment of cancer,

---

23. claims: 1-4, 6-15(all partially)

3-chloro-N-phenylaniline and derivatives, in particular as defined in claim 7 and enantiomers, diastereomers and pharmaceutically acceptable salts of any of the foregoing and pharmaceutical compositions thereof, for use as a medicament, in particular for the treatment of cancer,

---

24. claims: 1-4, 6-15(all partially)

1-(2-chlorophenyl)-3-phenylthiourea and derivatives, in particular as defined in claim 7 and enantiomers, diastereomers and pharmaceutically acceptable salts of any of the foregoing and pharmaceutical compositions thereof, for use as a medicament, in particular for the treatment of cancer,

---

25. claims: 1-4, 6-15(all partially)

2-(furan-2-ylmethyl)cyclopentanamine and derivatives, in particular as defined in claim 7 and enantiomers, diastereomers and pharmaceutically acceptable salts of any of the foregoing and pharmaceutical compositions thereof, for use as a medicament, in particular for the treatment of cancer,

---

## ANNEX TO THE EUROPEAN SEARCH REPORT
## ON EUROPEAN PATENT APPLICATION NO.

EP 12 17 0715

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2012

| Patent document cited in search report | | Publication date | Patent family member(s) | | Publication date |
|---|---|---|---|---|---|
| PT 1442050 | E | 31-01-2007 | AR | 037044 A1 | 20-10-2004 |
| | | | AT | 339439 T | 15-10-2006 |
| | | | AU | 2002351794 A2 | 12-05-2003 |
| | | | BR | 0213574 A | 26-10-2004 |
| | | | CA | 2464353 A1 | 08-05-2003 |
| | | | CN | 1578786 A | 09-02-2005 |
| | | | CO | 5580834 A2 | 30-11-2005 |
| | | | DE | 60214750 T2 | 13-09-2007 |
| | | | DK | 1442050 T3 | 08-01-2007 |
| | | | EG | 24335 A | 01-02-2009 |
| | | | EP | 1442050 A2 | 04-08-2004 |
| | | | ES | 2272792 T3 | 01-05-2007 |
| | | | HK | 1063326 A1 | 13-04-2007 |
| | | | HR | P20040360 A2 | 31-08-2004 |
| | | | HU | 0402081 A2 | 28-02-2005 |
| | | | JP | 4511835 B2 | 28-07-2010 |
| | | | JP | 2005521638 A | 21-07-2005 |
| | | | KR | 20050040815 A | 03-05-2005 |
| | | | MX | PA04003974 A | 25-01-2005 |
| | | | MY | 130373 A | 29-06-2007 |
| | | | NO | 331227 B1 | 07-11-2011 |
| | | | NZ | 533097 A | 29-07-2005 |
| | | | PA | 8557201 A1 | 30-06-2003 |
| | | | PE | 06112003 A1 | 02-09-2003 |
| | | | PL | 209411 B1 | 31-08-2011 |
| | | | PT | 1442050 E | 31-01-2007 |
| | | | RS | 36504 A | 10-04-2007 |
| | | | SI | 1442050 T1 | 30-04-2007 |
| | | | SV | 2004001370 A | 19-02-2004 |
| | | | UA | 78975 C2 | 10-05-2007 |
| | | | US | 2004259930 A1 | 23-12-2004 |
| | | | UY | 27514 A1 | 30-06-2003 |
| | | | WO | 03037916 A2 | 08-05-2003 |
| EP 0264109 | A1 | 20-04-1988 | DK | 538187 A | 16-04-1988 |
| | | | EP | 0264109 A1 | 20-04-1988 |
| | | | US | 4912100 A | 27-03-1990 |
| WO 8904319 | A1 | 18-05-1989 | EP | 0341318 A1 | 15-11-1989 |
| | | | WO | 8904319 A1 | 18-05-1989 |
| WO 2004089962 | A2 | 21-10-2004 | AU | 2004228478 A1 | 21-10-2004 |
| | | | CA | 2521384 A1 | 21-10-2004 |
| | | | CN | 1768069 A | 03-05-2006 |
| | | | EP | 1611143 A2 | 04-01-2006 |
| | | | JP | 2006522108 A | 28-09-2006 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 12 17 0715

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2012

| Patent document cited in search report | | Publication date | Patent family member(s) | | Publication date |
|---|---|---|---|---|---|
| | | | MX | PA05010736 A | 15-12-2005 |
| | | | US | 2006111328 A1 | 25-05-2006 |
| | | | WO | 2004089962 A2 | 21-10-2004 |
| EP 2206506 | A1 | 14-07-2010 | CN | 102256610 A | 23-11-2011 |
| | | | EP | 2206506 A1 | 14-07-2010 |
| | | | EP | 2373322 A1 | 12-10-2011 |
| | | | JP | 2012512828 A | 07-06-2012 |
| | | | US | 2011206650 A1 | 25-08-2011 |
| | | | WO | 2010069920 A1 | 24-06-2010 |
| WO 2011029054 | A1 | 10-03-2011 | AU | 2010289321 A1 | 05-04-2012 |
| | | | CA | 2773131 A1 | 10-03-2011 |
| | | | EP | 2473054 A1 | 11-07-2012 |
| | | | US | 2011065690 A1 | 17-03-2011 |
| | | | WO | 2011029054 A1 | 10-03-2011 |
| WO 2010138820 | A2 | 02-12-2010 | AU | 2010253820 A1 | 22-12-2011 |
| | | | CA | 2763589 A1 | 02-12-2010 |
| | | | EP | 2435040 A2 | 04-04-2012 |
| | | | US | 2012115915 A1 | 10-05-2012 |
| | | | WO | 2010138820 A2 | 02-12-2010 |
| WO 2009073620 | A2 | 11-06-2009 | CA | 2707308 A1 | 11-06-2009 |
| | | | CN | 101932325 A | 29-12-2010 |
| | | | EP | 2227233 A2 | 15-09-2010 |
| | | | JP | 2011505379 A | 24-02-2011 |
| | | | US | 2011053941 A1 | 03-03-2011 |
| | | | WO | 2009073620 A2 | 11-06-2009 |
| WO 2004006934 | A2 | 22-01-2004 | AU | 2003249999 A1 | 02-02-2004 |
| | | | WO | 2004006934 A2 | 22-01-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004006934 A

### Non-patent literature cited in the description

- **BOS, J.L.** *Cancer Res,* 1989, vol. 49, 4682-4689
- **CHUNG, S. et al.** *Nature Chem. Biol.,* 2009, vol. 5, 407-413
- **DALVIT, C. et al.** *J. Biomol. NMR,* 2001, vol. 21, 349-359
- **DOMINGUEZ, C. et al.** *J. Am. Chem. Soc.,* 2003, vol. 125, 1731-1737
- **GEYER, M. et al.** *Biochemistry,* 1996, vol. 35, 10308-10320
- **GRAF, T.** Entwicklung neuer Inhibitoren für das Ras-Protein und dessen onkogene Mutanten auf Basis von Cyclenderivaten. *PhD thesis,* 2006
- **HADJUK, P. J. ; GREER, J.** *Nature Rev.,* 2007, vol. 6, 211-219
- **KALBITZER H.R. et al.** *J. Am. Chem. Soc.,* 2009, vol. 131, 16714-16719
- **MAYER, M. ; MEYER B.** *Angew. Chem. Internat. Ed.,* 1999, vol. 38, 1784-1788
- **MAYER, M. ; MEYER B.** *J. Am. Chem. Soc.,* 2001, vol. 123, 6108-6117
- **NEUMANN, T. et al.** *Curr. Topics Med. Chem.,* 2007, vol. 7, 1630-1642
- **OPREA, T. I.** *J. Compuer-Aided Mol. Design,* 2000, vol. 14, 251-264
- **PALMER III, A.G. et al.** *J. Magn. Reson.,* 1991, vol. 93, 151-170
- **ROSNIZECK, I.C. et al.** *Angew. Chem. Int. Ed.,* 2010, vol. 49, 3830-3833
- **SCHANDA, P. ; BRUTSCHER B.** *J Am Chem Soc,* 2005, vol. 127, 8014-8015
- **SCHANDA, P. et al.** *J. Biomol. NMR,* 2005, vol. 33, 199-211
- **SCHLEUCHER, J. et al.** *J. Biomol. NMR,* 1994, vol. 4, 301-306
- **SCHMIDT, F. et al.** *Inorg. Chim. Acta,* 2011, vol. 365, 38-48
- **SCHUMANN, F.H. et al.** *J. Biomol. NMR,* 2007, vol. 39, 275-289
- **SHAKA, A.J. et al.** *J. Magn. Reson.,* 1985, vol. 64, 547-552
- **SPOERNER, M. et al.** *Proc. Natl. Acad. Sci. U.S.A.,* 2001, vol. 98, 4944-4949
- **SPOERNER, M. et al.** *FEBS J.,* 2007, vol. 274, 1419-1433
- **SPOERNER, M. et al.** *Biochemistry,* 2005, vol. 44, 2225-2236
- **SPOERNER, M. et al.** *Biochem. Biophys. Res. Commun.,* 2005, vol. 334, 709-713
- **SPOERNER, M. et al.** *J. Biol. Chem.,* 2010, vol. 285, 39768-39778
- **WITTINGHOFER, A. ; WALDMANN, H.** *Angew. Chem. Int. Ed.,* 2000, vol. 39, 4193-4214